(19) Europäisches Patentamt
European Patent Office
Office européen des brevets

(11) **EP 0 503 356 B1**

(12) **EUROPÄISCHE PATENTSCHRIFT**

(45) Veröffentlichungstag und Bekanntmachung des Hinweises auf die Patenterteilung:
**30.10.1996   Patentblatt 1996/44**

(51) Int Cl.[6]: **G01N 33/68**
// G01N33/49, G01N33/84,
G01N27/06, G01N27/333

(21) Anmeldenummer: **92103142.3**

(22) Anmeldetag: **25.02.1992**

(54) **Verfahren zur Bestimmung des Gesamteiweisses und des onkotischen Druckes eiweisshaltiger Körperflüssgkeiten**

Method for the determination of total protein and colloidosmotic pressure of protein containing body fluids

Procédé pour la détermination de protéine totale et de la pression colloidosmotique dans les fluides corporels contenant des protéines

(84) Benannte Vertragsstaaten:
**DE ES FR GB IT**

(30) Priorität: **09.03.1991  DE 4107689**

(43) Veröffentlichungstag der Anmeldung:
**16.09.1992   Patentblatt 1992/38**

(73) Patentinhaber: **Fresenius AG**
**D-61350 Bad Homburg (DE)**

(72) Erfinder:
• **Polaschegg, Hans-Dietrich, Dr.**
**W-6370 Oberursel 4 (DE)**
• **Westphal, Detlef, Dr.**
**W-6370 Oberursel 4 (DE)**
• **Metzner, Klaus**
**W-6382 Friedrichsdorf (DE)**

(74) Vertreter: **Fuchs, Luderschmidt & Partner**
**Patentanwälte**
**Postfach 46 60**
**65036 Wiesbaden (DE)**

(56) Entgegenhaltungen:
**DE-A- 3 627 814          DE-A- 4 025 425**

• **JOURNAL OF PHYSICAL CHEMISTRY, Band 42, Nr. 1, Januar 1938, Washington, DC (US); KEYS, Seiten 11-20**
• **IEEE TRANSACTIONS ON BIO-MEDICAL ENGINEERING, Band BME30, Nr. 3, März 1983, New York, NY (US); TRAUTMAN et al., Seiten 141-154**

**Beschreibung**

Die Erfindung betrifft ein Verfahren und eine Vorrichtung zur Bestimmung des Gesamteiweißes und des onkotischen Druckes eiweißhaltiger Körperflüssigkeiten.

Mehrere 100 Proteine kommen im menschlichen Serum bzw. Plasma vor. Die Plasmaproteine stellen eine sehr heterogene Gruppe von Körperproteinen dar, die im Gegensatz zu den intrazellulären Proteinen ihre Funktion im Extrazellulärraum (intravasaler und interstitieller Flüssigkeitsraum) ausüben.

Man unterscheidet:

- Transportproteine
  Albumin ist mit einer Konzentration von 40 - 52 g/l das quantitativ bedeutsamste Plasmaprotein, das ca. 60% der gesamten intravasalen Proteinmenge ausmacht. Es erfüllt zwei Hauptfunktionen:

    1. Aufrechterhaltung des kolloidosmotischen (onkotischen) Druckes, der im Plasma zu 80% vom Albumin bedingt wird.
    2. Albumin ist das wichtigste Transportprotein für Substanzen mit geringer Wasserlöslichkeit. Es bindet freie Fettsäuren, Bilirubin, ca 1/3 des Plasmacalciums, Spurenelemente und Hormone.
      Zu den Transportproteinen zählen weiterhin:
      Haptoglobin (mittl. Plasmakonzentration: 2 g/l)
      Transferrin (2.9 g/l)

- Immunglobuline
  Dazu gehören u.a. die folgenden Proteine (mit Angabe der Plasmakonzentration bei Erwachsenen):
  IgG (8 - 18 g/l), IgA (0.9 - 4.5 g/l), IgM (0.6 - 2.8 g/l)
- Enzyme
- Anti-Enzyme
- Proteohormone
    Die Gesamtkonzentration aller zu den 3 letzten Gruppen gehörenden Plasmaproteine ist kleiner als ca. 10 g/l.

Charakteristische Verschiebungen in der Zusammensetzung der einzelnen Eiweißfraktionen gibt es z. B. beim Auftreten von Nierenerkrankungen, Lebererkrankungen, akuten und chronischen Entzündungen, Tumoren und Antikörpermangelsyndromen.

Den Veränderungen der Gesamt-Protein-Konzentration im Plasma (Summe aller Plasmaproteine) liegen entweder Störungen des Wasser- und Elektrolythaushaltes zugrunde oder sie sind das Zeichen einer Dysproteinämie. Dysproteinämien sind Störungen der Plasmaproteinzusammensetzung aufgrund der Vermehrung, Verminderung oder dem Neuauftreten von Plasmaproteinen.

Absolute Veränderungen der Gesamt-Protein-Konzentration beruhen auch entweder auf einer Verminderung des Albumins oder der Zu- bzw. Abnahme der Immunglobuline. Die anderen Plasmaproteine unterliegen in ihrer Konzentration keinen den Referenzbereich der Gesamt-Protein-Konzentration verändernden Einflüssen.

Änderungen des Plasmavolumens (z.B. während der Dialysebehandlung) können hyper bzw. hypo-proteinämische Zustände verursachen, die daher auch als Pseudohyperproteinämie bzw. Pseudohypoproteinämie bezeichnet werden. Sie werden an einem der Gesamt-Protein-Konzentration gleichgerichteten Verhalten des Hämatokrit- und Hämoglobinwertes erkannt.

Ursachen der Pseudohypoproteinämie sind z.B. massive Blutungen, Infusionstherapie, Polydipsie, oder eine Schwangerschaft.

Erhöhungen der Gesamt-Protein-Konzentration beruhen entweder auf einer Vermehrung der Globuline oder auf einer Pseudohyperproteinämie. Ursachen der Pseudohyperproteinämien sind z.B. Dursten, Durchfälle, Erbrechen, polyurische Phase des akuten Nierenversagens und Diabetes insipidus.

Vor der Bestimmung der einzelnen Proteinfraktionen mit der Methode der Serum-Proteinelektrophorese zur Diagnostizierung einer Dysproteinämie ist es sinnvoll die Gesamt-Protein-Konzentration zu bestimmen, um Aussagen darüber machen zu können, ob überhaupt eine Erkrankung vorliegt.

Wie aus der Literatur (Keys,A.:The study of colloidal dimensions, thermodynamic activity, and the mean molecular weight of the mixed proteins in blood serum. J. phys. Chem.,42,11(1938), Ott,H.:Die Errechnung des kolloidosmotischen Serumdruckes aus dem Eiweiß-Spektrum und das mittlere Molekulargewicht der Serumeiweißfraktionen. Klin.Wschr.,34,1079(1956).) bekannt, kann aus den Konzentrationen der Plasmaproteine der kolloidosmotische Druck berechnet werden. Bei Vorliegen einer normalen Plasmaproteinverteilung (Euproteinämie) kann dieser Parameter auch aus der Gesamtproteinkonzentration ermittelt werden.

Der onkotische, auch onkodynamische bzw. kolloidosmotische Druck stellt einen Spezialfall des osmotischen Drucks dar. Hierbei handelt es sich allgemein um denjenigen Druck, den man aufbringen müßte, um das Bestreben eines Lösungsmittels, durch eine semipermeable Membran in eine Lösung hineinzufließen, gerade zum Stillstand zu bringen.

Der onkotische Druck spielt eine große Rolle für das Wasserbindungsvermögen von Geweben und Körperflüssigkeiten, insbesondere Blutplasma, sowie die Wasserausscheidung in den Nieren.

Einen besonderen Einfluß hat der onkotische Druck auf die Flüssigkeitsverteilung zwischen Vasalraum und interstitiellem Raum. So bestimmt beispielsweise die Größe der Differenz aus onkotischem Druck und Blutdruck die Größe des Flüssigkeitsaustausches zwischen Gefäßen und Geweben. Je größer der Blutdruck ist, beispielsweise durch Stauung, desto mehr Übertritt in das Gewebe findet statt. Es kann zu Lymphansammlungen oder Ödemen kommen. Je größer der onkotische Druck ist, desto mehr Flüssigkeitsrückstrom in die Kappillaren findet statt.

Unter klinischen Aspekten ist der onkotische bzw. kolloidosmotische Druck ein wichtiger physiologischer Parameter für die Beurteilung des Zustandes eines Patienten in der Intensivmedizin, beispielsweise bei Operationen am offenen Herzen oder auch bei der Dialysebehandlung. Dabei werden zur Aufrechterhaltung des onkotischen Drucks entweder körpereigene Blutbestandteile (Blutplasma, Albumin etc.) oder körperfremde Plasmaexpander per Infusion appliziert.

Der kolloidosmotische Effekt kann direkt durch die Druckdifferenz an einer semipermeablen Membran bestimmt werden, die Lösung und Lösungsmittel trennt. Durch Messung einer Standard- Albuminlösung wird eine Kalibrierung durchgeführt (Schröck,R.:Dissertation: Zur Methodik der Messung des kolloidosmotischen Druckes biologischer Flüssigkeiten.München,1974.).

Die folgenden Methoden zur quantitativen Bestimmung des Gesamt-Proteins sind bekannt (Boroviczeny, K.G.v.,(Hrsg):Qualitätssicherung im Medizinischen Laboratorium.Springer Verlag,1987):

- Farbkomplexbildung von zugesetzten Substanzen mit dem Protein (Photometrie; Biuret-Reaktion)
- Trübungsmessungen des gefällten Proteins nach Zusatz von Fällungsmitteln (Turbidimetrie)
- Messung des im Protein enthaltenen Stickstoffs (Rest-Stickstoff-Bestimmung nach Kjeldahl).

Die Gesamt-Protein-Bestimmung erfolgt am häufigsten mit der Biuret-Methode. Das Prinzip der Biuret-Methode beruht auf der Anlagerung von Kupferionen im alkalischen pH-Wert-Bereich an die Peptidbindungen von Proteinen und Peptiden. Die Intensität der dabei entstehenden Violettverfärbung ist proportional der Zahl der Peptidbindungen und damit der Proteinkonzentration in einem weiten Bereich.

Ein Nachteil der Biuret-Methode ist die lange Wartezeit von 45 min bis sich der Farbkomplex ausgebildet hat.

Schon Ende des 19. Jahrhunderts wurde versucht durch Messung der elektrischen Leitfähigkeit von physiologischen Flüssigkeiten Informationen über die Zusammensetzung der Elektrolyte und Nichtelektrolyte zu erhalten. Arrhenius wies nach, daß die Leitfähigkeit durch Zusatz von Nichtleitern vermindert wird. Die Nichtleiter des Serums sind dessen Eiweißkörper und die übrigen organischen Verbindungen (Bugarszky,St., Tangl,F.:Physikalisch-chemische Untersuchungen über die molekularen Concentrationsverhältnisse des Blutserums.Arch.für Physiol., Jg.72,1898).

Aus der DE-PS 36 27 814 ist eine Vorrichtung zur Bestimmung des Hämatokritwerts aufgrund von Leitfähigkeitsmessungen bekannt. Hierbei wird die Leitfähigkeit von Vollblut und die Leitfähigkeit von Blutplasma bestimmt und aus den gewonnenen Leitfähigkeitswerten der Hämatokrit ermittelt. Die Plasmaleitfähigkeit wird hierbei aus der im Blut vorliegenden Natrium- und Kaliumionenkonzentration ermittelt.

Gegenüber dem diskutierten Stand der Technik liegt der Erfindung die Aufgabe zugrunde, ein Verfahren bereitzustellen, mit dem das Gesamteiweiß und daraus der onkotische Druck nicht nur relativ einfach und schnell, sondern auch kontinuierlich bestimmt werden kann.

Die Lösung der Aufgaben erfolgt durch ein Verfahren mit den Merkmalen des Anspruchs 1, gegebenen falls unter Verwendung einer Vorrichtung mit den Kennzeichen des Anspruchs 7.

Beim Verfahren der in Rede stehenden Erfindung erhält man den Elektrolytleitfähigkeitswert in der Weise, daß man die im Plasma vorliegende Natriumionen Konzentration $c_{Na}$ oder die Chloridionen Konzentration $c_{Cl}$ mißt und aus den erhaltenen Konzentrationswerten die Elektrolytleitfähigkeit gemäß der Gleichung

a)

$$LF_{Elek} = A * c_{Na} + H$$

oder

b)

$$LF_{Elek} = F * c_{Cl} + G$$

errechnet, wobei die Konstanten A, H, F, G mittels eines an sich bekannten Leitfähigkeitsmeßverfahrens zuvor ermittelt werden.

Nachdem die Leitfähigkeit der Plasmaprobe $LF_P$ bestimmt wurder wird aus der Plasmaleitfähigkeit ($LF_P$) und der Elektrolytleitfähigkeit ($LF_{Elek}$) die Gesamtproteinkonzentration errechnet.

Das erfindungsgemäße Verfahren weist zunächst den Vorteil auf, daß Plasma bzw Serum direkt sowohl zur Bestimmung des Plasmaleitfähigkeitswertes als auch zur Bestimmung der Elektrolytleitfähigkeit des Plasmas eingesetzt werden kann. Die Elektrolytleitfähigkeit des Plasmas ist dabei die Leitfähigkeit, die eine Lösung gleicher Elektrolytzusammensetzung aber ohne Anwesenheit der Serumproteine aufweist (Entspricht dem theoretischen Leitfähigkeitswert des Ultrafiltrats einer Serumprobe bei Kompensation der durch den Donnan-Effekt verursachten Elektrolytverschiebung).

Dabei wird die Elektrolytleitfähigkeit des Plasmas im wesentlichen durch das Natriumion oder das Chloridion gebildet, wobei das Natriumion zu etwa 96% den Leitfähigkeitswert bildet und das Kaliumion sowie die übrigen Kationen den Restwert von etwa 4% darstellen.

Diese Angaben beziehen sich auf die hier behandelten Änderungen der Leitfähigkeit aufgrund physiologischer Schwankungen der Elektrolytzusammensetzung. Wenn man den Leitfähigkeitsbeitrag der Bicarbonat-Anionen als im wesentlichen konstant ansieht (Änderungen der Bicarbonat-Konzentration von +- 5 mmol/ l führen zu einer Verfälschung des Gesamt-Proteins von ca. +- 4%), ist die Änderung der Kationen korreliert mit einer entsprechenden Änderung der Chlorid-Anionen.

Der Einsatz von ionenselektiven Elektroden ist insofern vorteilhaft, als durch diese Elektroden die Konzentration der Elektrolyte bestimmt wird, die in erster Näherung von dem Gesamt-Proteingehalt der Probe unabhängig sind. Die sich hieraus ergebende Konzentration kann mit der konzentrationsabhängigen molaren Leitfähigkeit des jeweiligen Elektrolyten (z.B. NaCl) multipliziert zur spezifischen Elektrolytleitfähigkeit der Plasmaprobe umgerechnet werden.

Weitere vorteilhafte Modifikationen des erfindungsgemäßen Verfahrens werden in den abhängigen Ansprüchen 2 - 6 unter Schutz gestellt.

Zusätzlich zu der natriumselektiven Elektrode kann eine kaliumselektive Elektrode zur Bestimmung der Kaliumionen Konzentration $c_K$ eingesetzt werden, um die Gesamtelektrolytleitfähigkeit des Plasmas exakter zu bestimmen. Die Elektrolytleitfähigkeit des Plasmas kann dann entsprechend der linearen Gleichung

$$LF_{Elek} = A * c_{Na} + B * c_K + C$$

errechnet werden, wobei B und C Konstanten sind, die entsprechend dem erfindungsgemäßen Verfahren zu ermitteln sind.

In einer weiteren Ausführungsform kann man zusätzlich weitere Kationen - und Anionenkonzentrationen, beispielsweise die Calcium-, Magnesium und Bicarbonationenkonzentration messen und daraus die Elektrolytleitfähigkeit des Plasmas errechnen. Insbesondere bevorzugt ist die Verwendung einer Bicarbonat-Elektrode, um auch Aufschluß über die Zusammensetzung der Anionen zu erhalten.

Diese ionenselektiven Elektroden werden vorteilhafterweise in einer einzigen Vorrichtung zusammen mit einer Leitfähigkeitsmeßeinrichtung vorgesehen, so daß sowohl die in einer Plasmaprobe enthaltenen Elektrolyte als auch die Leitfähigkeit der Plasmaprobe nebeneinander bestimmt werden können, ohne daß die Abtrennung von Ultrafiltrat aus dem Plasma mittels eines geeigneten Filters notwendig wäre.

Besonders vorteilhaft wird eine Durchflußeinrichtung eingesetzt, die einen Durchflußkanal aufweist, in den die ionenselektiven Elektroden und die Leitfähigkeitsmeßzelle eingeschaltet sind. In einen solchen Durchflußkanal kann mit Hilfe z.B. einer zusätzlichen Pumpe die Plasmaprobe gefördert werden, oder aber die Durchflußmeßeinrichtung wird über einen Plasmafilter, der in einen extrakorporalen Blutkreislauf integriert ist, mittels einer zusätzlichen Pumpe kontinuierlich mit Plasmaprobe versorgt. So kann einem extrakorporalen Blutkreislauf, der beispielsweise bei der Hämodialyse aufgebaut wird, kontinuierlich Plasma entzogen werden, das in einem derartigen Durchflußanalysator analysiert wird.

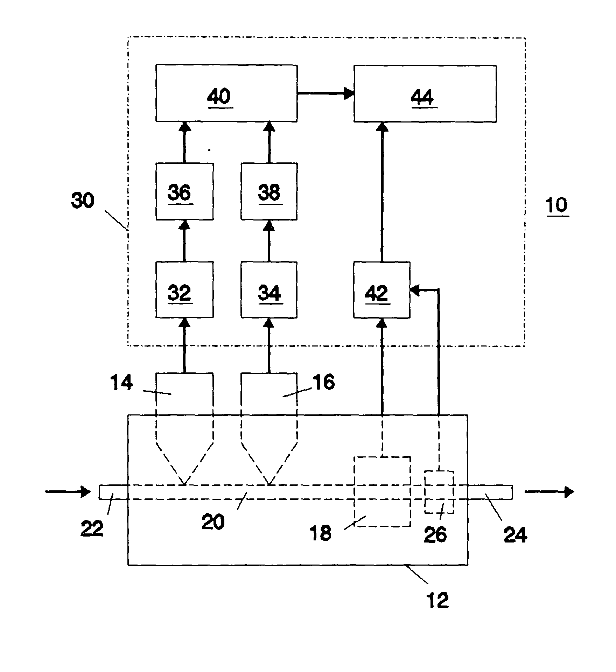

Weitere Vorteile, Merkmale und Einzelheiten werden anhand eines Ausführungsbeispiels unter Bezug auf die Zeichnung erläutert.

Die Figur zeigt eine Prinzipskizze einer Durchflußanordnung mit zwei ionenselektiven Elektroden und einer Leitfähigkeitszelle gemäß der Erfindung.

In der Figur ist die Vorrichtung zur Bestimmung des Gesamt-Proteins mit 10 bezeichnet. Diese Vorrichtung 10 weist ein Gehäuse 12 auf, in dem eine natriumionenselektive Elektrode 14, eine kaliumionenselektive Elektrode 16 und eine Leitfähigkeitszelle 18 untergebracht sind, wobei die Elektroden 14 und 16 in an sich bekannter Weise mit einer nichtgezeigten Referenzelektrodenanordnung verbunden sind. Des weiteren weist das Gehäuse 12 einen Durchflußkanal 20 auf, der mit einem Eingangsstutzen 22 und einem Ausgangsstutzen 24 in Verbindung ist.

Mit diesem Durchflußkanal 20 sind die natriumionenselektive Elektrode 14, die kaliumionenselektive Elektrode 16 und die Leitfähigkeitszelle 18 derart verbunden, daß das durch den Durchflußkanal 20 zu führende Plasma mit ihnen in Berührung und somit jeweils ein Meßsignal auslösen kann.

Derartige Durchflußanordnungen sind beispielsweise aus der DE PS 34 16 956 bekannt. Insofern kann auf weitere Einzelheiten der ionenselektiven Elektroden, die im übrigen bekannt sind, verzichtet werden. Des weiteren sind auch Leitfähigkeitsmeßzellen allgemein bekannt.

Plasma kann mit Hilfe einer nichtgezeigten Zuführungseinrichtung dem Durchflußkanal 20 zugeführt und durch diesen gefördert werden. Zur Messung verbleibt die Plasmaprobe stationär im Durchflußkanal 20 und wird anschließend in einem Spülvorgang aus dem Durchflußkanal mit einem Spülfluid verdrängt.

Das Gehäuse 12 kann weiterhin eine nichtgezeigte Thermostateinrichtung oder eine am Durchflußkanal angeordnete Temperaturmeßeinrichtung 26 aufweisen, um Temperatureffekte durch Stabilisierung bzw. rechnerisch zu kompensieren. Somit kann auch die Vorrichtung 10 thermisch mit der Umgebung im Gleichgewicht sein, ohne daß eine Thermostatisierung notwendig wäre.

Die natriumionenselektive Elektrode 14, die kaliumionenselektive Elektrode 16 und die Leitfähigkeitszelle 18 sind mit einem Rechner 30 verbunden, der strichliert in der Figur dargestellt ist. Dieser Rechner 30 weist die nachstehend beschriebenen Einheiten auf, mit denen die Gesamt-Protein-Konzentration und der Onkotische Druck aus den von den Elektroden und der Leitfähigkeitszelle abgegebenen Signalen errechnet werden kann.

In der Recheneinheit 32 bzw. 34 wird zunächst aus dem Signal der natriumionenselektiven Elektrode 14 bzw. der kaliumionenselektiven Elektrode 16 die Natriumionenkonzentration $c_{Na}$ bzw. die Kaliumionenkonzentration $c_K$ errechnet. Hierzu werden die ionenselektiven Elektroden nach einem bekannten Verfahren mit Normlösungen kalibriert, wobei die Recheneinheiten 32 und 34 zur Errechnung der Ionenkonzentrationen die gemessenen Signale mit Referenzsignalen vergleichen und somit die tatsächliche, im Plasma enthaltene Ionenkonzentration ermitteln.

Den in den Recheneinheiten 32 und 34 ermittelten Konzentrationswerten wird in den Zuordnungseinheiten 36 und 38 eine empirische Konstante A bzw. B zugeordnet, so daß sich hieraus für die Natriumionenkonzentrationen das Produkt $A * c_{Na}$ und für die Kaliumionenkonzentrationen das Produkt $B * c_K$ ergeben. Die empirischen Größen A und B werden zuvor experimentell bestimmt und stellen somit Fitgrößen dar. Sie enthalten die molaren Leitfähigkeiten der Natrium- bzw. Kaliumionen im entsprechenden Konzentrationsbereich.

Die in den Zuordnungseinheiten 36 und 38 ermittelten Produkte stellen somit die spezifische Leitfähigkeit der jeweiligen Natrium- und Kaliumionen in der Plasmaprobe dar, zu denen zusätzlich noch eine Konstante C hinzuzufügen ist, die die restlichen im Plasma enthaltenen Kationen und Anionen berücksichtigt. Hierzu werden die in den Einheiten 36 und 38 ermittelten Werte in einem Summierer 40 zusammen mit der Konstante C zu der spezifischen Elektrolytleitfähigkeit der Plasmaprobe addiert, wie dies aus Gleichung (1) ersichtlich ist.

$$LF_{Elek} = A * c_{Na} + H \qquad (1)$$

$$LF_{Elek} = A * c_{Na} + B * c_K + C \qquad (2)$$

wobei

$$H = B * c_K + C$$

ist

Die Leitfähigkeitsmeßzelle 18 und ggf. die Temperaturmeßeinrichtung 26 sind mit einer Einheit 42 zur Bestimmung der spezifischen Leitfähigkeit der Plasmaprobe $LF_P$ verbunden, wobei der ermittelte Wert ggf. temperaturkompensiert werden kann. Diese Einheit 42 errechnet aus den übertragenen Signalen den spezifischen Leitfähigkeitswert und gibt den ermittelten Wert an eine Einheit 44 weiter, in der aus folgender Gleichung (3)

$$c_{TProt} = D * ( LF_{Elek} - LF_P ) + E \qquad (3)$$

die Gesamt-Protein-Konzentration ermittelt wird, wobei die Konstanten D und E empirische Fitkonstanten sind. Üblicherweise werden sämtliche vorstehend genannten Konstanten A, B, C, D, E und H anhand einer Korrelationsmessung mit bekannten Werten für die Gesamt-Protein-Konzentration normiert, die sich beispielsweise aus Messungen nach der Biuret-Methode ergeben.

Diese Einheit 44 wird weiterhin mit dem in dem Summierer 40 ermittelten spezifischen Leitfähigkeitswert gespeist und ermittelt so nach der vorstehenden Gleichung die Gesamt-Protein-Konzentration. An diese Einheit ist eine übliche, nicht gezeigte Anzeige oder eine sonstige Einrichtung zur Speicherung des ermittelten Werts angeschlossen.

Es hat sich gezeigt, daß der mit dieser Vorrichtung ermittelte Gesamt-Protein-Wert in Übereinstimmung steht mit dem mittels der Biuret-Methode gemessenem Wert. Die oben genannte lineare Beziehung für Gesamt-Protein kann für den gesamten physiologischen Bereich (20 - 150 g/l) angepaßt werden.

In gleicher Weise wie zur Bestimmung der Natriumionenkonzentration kann mit einer chloridionenselektiven Elektrode der Chloridionengehalt von Plasma bestimmt werden, aus dem sich entsprechend der Gleichung (4)

$$LF_{Elek} = F * c_{Cl} + G \qquad (4)$$

die Elektrolytleitfähigkeit von Plasma berechnet, wobei die Konstanten F und G wiederum wie die vorstehend genannten Konstanten empirische Konstanten sind.

Es hat sich gezeigt, daß brauchbare Gesamt-Protein-Werte mit folgenden Werten erhalten werden, wobei die Konzentration in Mol/l, die spezifische Leitfähigkeit in mS/cm, die molare Leitfähigkeit in $(mS/mol)*cm^2$ und die Gesamt-Protein-Konzentration in g/l angegeben werden.

Die Konstante A liegt in einem Bereich von 95 - 115, vorzugsweise etwa 105, die Konstante B liegt in einem Bereich von 104 - 138, vorzugsweise 126, die Konstante C liegt in einem Bereich von 0 - -1, vorzugsweise -0,5, die Konstante D liegt in einem Bereich von 21 - 27, vorzugsweise 24 und die Konstante E liegt in einem Bereich von -1 - -3, vorzugsweise -2. Die mit diesen Werten ermittelten Gesamt-Protein-Konzentrationen stimmen mit den Werten gemäß den üblichen Methoden zur Bestimmung des Gesamt-Proteins innerhalb von etwa 4% überein, wobei diese Konstanten besonders vorteilhaft in einem Gesamt-Proteinbereich von 40 - 120 g/l einsetzbar sind.

Mit einer aus der Literatur bekannten Umrechnungsformel (Keys,A.:The study of colloidal dimensions, thermodynamic activity, and the mean molecular weight of the mixed proteins in blood serum. J.phys. Chem.,42, 11(1938), Ott,H.:Die Errechnung des kolloidosmotischen Serumdruckes aus dem Eiweiß-Spektrum und das mittlere Molekulargewicht der Serumeiweißfraktionen. Klin. Wschr.,34,1079(1956).) kann in der Einheit 44 zusätzlich aus der Gesamt-Protein-Konzentration der Onkotische bzw. kolloidosmotische Druck berechnet werden.

## Patentansprüche

1. Verfahren zur Bestimmung der Gesamteiweißkonzentration proteinhaltiger Körperflüssigkeiten aus der Leitfähigkeit ($LF_P$) und der Elektrolytleitfähigkeit ($LF_{Elek}$) einer Blutplasma- oder Serumprobe, wobei man den Elektrolytleitfähigkeitswert ($LF_{Elek}$) in der Weise erhält, daß man die im Plasma vorliegende Natriumionenkonzentration $c_{Na}$ oder die Chloridionenkonzentration $c_{Cl}$ mißt und aus den erhaltenen Konzentrationswerten die Elektrolytleitfähigkeit gemäß der Gleichung

   a)

   $$LF_{Elek} = A * c_{Na} + H$$

   oder
   b)

   $$LF_{Elek} = F * c_{Cl} + G$$

   errechnet, wobei die Konstanten A, H, F, G mittels eines an sich bekannten Leitfähigkeitsmeßgerätes zuvor ermittelt werden,

   anschließend die Leitfähigkeit der Plasmaprobe $LF_P$ bestimmt und

   die Gesamtproteinkonzentration aus der Plasmaleitfähigkeit ($LF_P$) und der Elektrolytleitfähigkeit ($LF_{Elek}$) entsprechend der Gleichung

   $$C_{TProt} = D * (LF_{Elek} = LF_P) + E$$

   errechnet, wobei die Konstanten D und E mittels eines an sich bekannten Gesamtproteinmeßverfahrens zuvor ermittelt wurden.

2. Verfahren nach Anspruch 1, dadurch gekennzeichnet, daß man neben der Natriumionenkonzentration die Kaliumionenkonzentration $c_K$ bestimmt.

3. Verfahren nach Anspruch 2, dadurch gekennzeichnet, daß man die Elektrolytleitfähigkeit von Plasma entsprechend der linearen Gleichung

   $$LF_{Elek} = A * c_{Na} + B * c_K + C$$

errechnet , wobei B und C Konstanten sind, die entsprechend dem Verfahren von Anspruch 1 ermittelt wurden.

4. Verfahren nach Anspruch 2, dadurch gekennzeichnet, daß man zusätzlich weitere Kationen- und Anionenkonzentrationen, beispielsweise die Calcium-, Magnesium und Bicarbonationenkonzentration mißt und daraus die Elektrolytleitfähigkeit des Plasmas errechnet.

5. Verfahren nach Anspruch 1, dadurch gekennzeichnet daß nach einer an sich bekannten Umrechnungsformel aus der Gesamt-Protein-Konzentration der onkotische Druck berechnet wird.

6. Verfahren nach einem der Ansprüche 1 bis 5, dadurch gekennzeichnet, daß man die Ionenkonzentrationen mit ionenselektiven Elektroden mißt.

7. Verwendung einer Vorrichtung mit einer Einrichtung zur Messung der Leitfähigkeit, einer natriumionenselektiven Elektrode sowie einer Recheneinheit zur Ermittlung der Elektrolytleitfähigkeit des Plasmas aus dem Signal der natriumionenselektiven Elektrode, wobei die Recheneinheit eine Einheit zur Bestimmung der Natriumionenkonzentration aus dem Signal der natriumselektiven Elektrode, eine Zuordnungseinheit die den in der Einheit ermittelten Konzentrationswert mit einer Konstanten versieht und einen Summierer aufweist, mit dem der in der Zuordnungseinheit ermittelte Wert mit einer weiteren Konstanten addiert wird, wobei die Recheneinheit (30) eine weitere Einheit (44) zur Ermittlung der Gesamt-Protein-Konzentration und des Onkotischen Drucks aus der vom Summierer (40) abgegebenen Elektrolytleitfähigkeit des Plasmas und des von der Einrichtung (42) abgegebenen Leitfähigkeitswertes des Plasmas aufweist, zur Durchführung des Verfahrens gemäß Ansprüchen 1-6.

8. Verwendung nach Anspruch 7, dadurch gekennzeichnet, daß sich neben der natriumionenselektiven Elektrode (14) zusätzlich eine kaliumionenselektive Elektrode (16) befindet.

9. Verwendung nach Anspruch 7 und 8, dadurch gekennzeichnet, daß sich die ionenselektiven Elektroden (14,16) und die Leitfähigkeitszelle (18) in einem einen Durchflußkanal (20) aufweisenden Gehäuse (12) befinden.

## Claims

1. Method to determine the total albumin concentration of protein containing bodily fluids by determining the conductivity value ($Cond._P$) and the electrolyte conductivity value of a blood plasma or serum sample, by which the electrolyte conductivity value (Cond.$_{Elec}$) is obtained in a manner such that the sodium ion concentration $C_{Na}$ or the chloride ion concentration $C_{Cl}$ in plasma is measured, and the electrolyte conductivity is calculated from the concentration values obtained according to the equation

a)

$$Cond._{Elec} = A * C_{Na} + H$$

or
b)

$$Cond._{Elec} = F * C_{Cl} + G,$$

whereby the constants A, H, F, G are first determined by means of a conductivity measurement method that is known per se,

whereby subsequently the conductivity of the plasma sample (LF$_P$) is determined and

the total protein concentration is calculated from the conductivity (Cond.$_P$) of the plasma and the electrolyte conductivity (Cond.$_{Elec}$) according to the equation

$$C_{TProt} = D * (Cond._{Elec} - Cond._P) + E,$$

whereby the constants D and E are determined by means of a total protein measuring method that is known per se.

2. Method according to claim 1, characterized in that, in addition to the sodium ion concentration, the potassium ion concentration $C_K$ is determined.

3. Method according to claim 2, characterized in that the electrolyte conductivity in the plasma is calculated according to the linear equation

$$Cond._{Elec} = A * C_{Na} + B * C_K + C$$

whereby B and C are constants that are determined according to the method of claim 1.

4. Method according to claim 2, characterized in that other, additional cation and anion concentrations, for example calcium, magnesium and bicarbonate concentrations, are measured and the electrolyte conductivity of plasma calculated therefrom.

5. Method according to claim 1, characterized in that the oncotic pressure is determined according to a conversion formula known per se, from the total protein concentration.

6. Method according to one of the claims 1 to 5, characterized in that the ion concentrations are measured with selective ion electrodes.

7. Use of a device with a device to measure the conductivity, a selective sodium electrode and a computer to determine the electrolyte conductivity of the plasma from the selective sodium electrode signal, whereby the computer unit features a unit to determine the sodium ion concentration from the selective sodium electrode signal, an allocation unit which provides the concentration value determined in the unit with a constant, and a summation device, with which the value determined in the allocation unit is added to another constant, whereby the computer unit (30) features another unit (44) to determine the total protein concentration and the oncotic pressure from the plasma electrolyte conductivity generated by summation device (40) and the plasma conductivity value generated by the device (42) to carry out the method according to claims 1 to 6.

8. Use according to claim 7, characterized in that, in addtion to the selective sodium electrode (14) there is also a selective potassium electrode (16).

9. Use according to claim 7 and 8, characterized in that the selective ion electrodes (14,16) and the conductivity cell (18) are located in housing (12) that features a flow channel (20).

**Revendications**

1. Procédé pour déterminer la concentration en protéine totale dans les fluides corporels contenant des protéines, à partir de la conductivité (LF$_P$) et de la conductivité électrolytique (LF$_{élec}$) d'un échantillon de plasma sanguin ou de sérum, en obtenant la valeur de la conductivité électrolytique (LF$_{élec}$) de la façon telle, qu'on mesure la concentration en ions sodium $c_{Na}$ ou la concentration en ions chlorure $c_{Cl}$ présente dans le plasma, et on calcule à partir des valeurs de la concentration la conductivité électrolytique selon l'équation

a)

$$LF_{élec} = A * c_{Na} + H$$

ou
b)

$$LF_{\text{élec}} = F * c_{Cl} + G$$

où on détermine les constantes A, H, F, G à l'aide d'un dispositif de mesures de la conductivité connu en soi, ensuite on détermine la conductivité de l'échantillon de plasma $LF_p$ et la concentration en protéine totale à partir de la conductivité du plasma ($LF_P$) et de la conductivité électrolytique ($LF_{\text{élec}}$) conformément à l'équation

$$C_{TProt} = D * (LF_{\text{élec}} - LF_P) + E$$

en déterminant auparavant les constantes D et E à l'aide d'un procédé de mesures de la protéine totale connu en soi.

2.  Procédé selon la revendication 1, caractérisé en ce qu'on détermine outre la concentration en ions sodium, la concentration en ions potassium.

3.  Procédé selon la revendication 2, caractérisé en ce qu'on calcule la conductivité électrolytique du plasma selon l'équation linéaire

$$LF_{\text{élec}} = A * c_{Na} + B * c_K + C$$

B et C étant des constantes que l'on détermine conformément au procédé de la revendication 1.

4.  Procédé selon la revendication 2, caractérisé en ce qu'on mesure de plus les concentrations en d'autres cations et anions, par exemple les concentrations en ions calcium, magnésium et bicarbonate, et à partir de celles-ci, on calcule la conductivité électrolytique du plasma.

5.  Procédé selon la revendication 1, caractérisé en ce qu'on calcule la pression oncotique à partir de la concentration en protéine totale à l'aide d'une formule de calcul connue en soi.

6.  Procédé selon l'une des revendications 1 à 5, caractérisé en ce qu'on mesure les concentrations en ions à l'aide d'électrodes sélectives d'ions.

7.  Utilisation d'un dispositif avec une installation pour la mesure de la conductivité, d'une électrode sélective d'ions sodium, ainsi que d'une unité de calcul pour déterminer la conductivité électrolytique du plasma à partir du signal de l'électrode sélective d'ions sodium, l'unité de calcul étant une unité pour la détermination de la concentration en ions sodium à partir du signal de l'électrode sélective de sodium, d'une unité de référence qui pourvoit d'une constante la valeur de la concentration déterminée dans l'unité, et qui présente un additioneur analogique, à l'aide duquel on additione la valeur déterminée dans l'unité de référence avec une autre constante, l'unité de calcul (30) présentant une autre unité (44) pour la détermination de la concentration en protéine totale et de la pression osmotique à partir de la conductivité électrolytique du plasma transmise par l'additioneur analogique (40) et de la valeur de conductivité transmise par l'installation (42) pour la mise en oeuvre du procédé selon les revendications 1 à 6.

8.  Utilisation selon la revendication 7, caractérisée en ce qu'aux côtés de l'électrode sélective d'ions sodium (14), on trouve de plus une électrode sélective d'ions potassium (16).

9.  Utilisation selon la revendication 7 et 8, caractérisée en ce que les électrodes sélectives d'ions (14) (16) et la cellule de conductivité (18) se trouvent dans un boîtier (12) présentant un canal d'écoulement (20).